# EUROPEAN PATENT SPECIFICATION

(11) **EP 3 970 754 B1**
(45) Date of publication and mention of the grant of the patent: **14.10.2026**
(21) Application number: 20805903.0
(22) Date of filing: 14.05.2020
(51) Int. Cl.: A61K 35/761, A61K 35/763, A61K 35/768, A61K 35/76, A61P 35/00, A61K 48/00, A61M 25/00

(54) **ONCOLYTIC VIRUS FOR USE IN THE TREATMENT OF A TUMOR USING AN ENDOSCOPE**
ONKOLYTISCHES VIRUS ZUR VERWENDUNG BEI DER BEHANDLUNG EINES TUMORS MITTELS EINES ENDOSKOP
VIRUS ONCOLYTIQUE POUR UTILISATION DANS LE TRAITEMENT D'UNE TUMEUR À L'AIDE D'UN ENDOSCOPE

(30) Priority: 14.05.2019 US 201962847412 P
(43) Date of publication of application: 23.03.2022
(73) Proprietor: Oncolys BioPharma, Inc., Tokyo 105-0001 (JP)
(72) Inventor: URATA, Yasuo, Tokyo 105-0001 (JP); SUDA, Hiroyuki, Tokyo 105-0001 (JP); SHOJI, Koichiro, Tokyo 105-0001 (JP)
(74) Representative: J A Kemp LLP
(86) International application number: PCT/JP2020/019211
(87) International publication number: WO 2020/230838

(56) References cited:
- EP-A1- 1 553 178
- WO-A1-2016/174200
- JP-A- 2016 532 496
- US-A1- 2011 044 949
- SHIRLEY LAWRENCE A. ET AL: "Therapeutic Endoscopic Ultrasonography: Intratumoral Injection for Pancreatic Adenocarcinoma", vol. 2013, 1 January 2013 (2013-01-01), us, pages 1 - 3, XP093054541, ISSN: 1687-6121, Retrieved from the Internet <URL:https://www.ncbi.nlm.nih.gov/pmc/articles/PMC3623468/pdf/GRP2013-207129.pdf> DOI: 10.1155/2013/207129
- KAGAWA, S. ET AL.: "A phase I/II study of endoscopic intratumoral administration of a telomerase targeted oncolytic virus, OBP-301, in combination with radiotherapy for esophageal cancer", ANNUAL MEETING OF JAPAN SOCIETY OF CLINICAL ONCOLOGY, vol. 53, pages 1 - 4, XP055760388
- TAKESHI KOUJIMA; HIROSHI TAZAWA; NAOTO HORI; SHUTA TAMURA; SHINJI KURODA; HIROYUKI KISHIMOTO; MASAHIKO NISHIZAKI; YASUO URATA; SHU: "A novel tumor-specific oncolytic biological therapy against invasive pancreatic cancer", CANCER RESEARCH, vol. 75, no. 15, 1 August 2015 (2015-08-01), pages 1 - 4, XP009524635, DOI: 10.1158/1538-7445.AM2015-3535
- HIROOKA, Y. ET AL.: "A Phase I clinical trial of EUS-guided intratumoral injection of the oncolytic virus, HF10 for unresectable locally advanced pancreatic cancer", BMC CANCER, vol. 18, no. 1, 2018, pages 1 - 9, XP055760408

## Description

### Technical Field

The present invention relates to a virus for use in a method of treating a tumor using an endoscope, wherein the method comprises inserting an endoscopic puncture needle filled with a virus-containing solution into tumor tissues and injecting the virus into the tumor tissues, and wherein the virus is administered to the peripheral base of the tumor and/or the marginal region of the tumor tissues, and wherein the virus is an oncolytic virus.

### Background Art

Oncolytic virus has been proposed as a tool for developing novel strategies for cancer therapy, and in preclinical researches and clinical trials, the effects of the oncolytic virus in the clinical situation have been studied. As such oncolytic viruses, several viruses, such as adenovirus, herpesvirus, vesicular stomatitis virus, reovirus, vaccinia virus and measles virus, have been reported. The antitumor ability of an oncolytic adenovirus has been demonstrated according to preclinical tests and clinical trials.

OBP-301 is an oncolytic adenovirus that has been modified by introducing a human telomerase reverse transcriptase (hTERT) promotor into the genome thereof, so that the gene can selectively replicate in cancer cells, and it is expected that such OBP-301 will be used to treat various types of cancers. Lawrence et al. (Non Patent Literature 2) reports therapeutic endoscopic ultrasonography, in particular intratumoral injection for pancreatic adenocarcinoma. WO 2016/174200 A1 (Patent Literature 1) discloses a specific oncolytic adenovirus encoding a B7 protein. US 2011/044949 A1 (Patent Literature 2) discloses a specific oncolytic virus replicating selectively in tumour cells. Kagawa, S. et al. (Non Patent Literature 3) report a phase I/II study of endoscopic intratumoral administration of a telomerase targeted oncolytic virus, OBP-301, in combination with radiotherapy for esophageal cancer. Koujima et al. (Non Patent Literature 4) reports a novel tumor-specific oncolytic biological therapy against invasive pancreatic cancer.

### Prior Art Documents

### Patent Literature

Patent Literature 1: WO 2016/174200 A1
Patent Literature 2: US 2011/044949 A1

### Non Patent Literature

Non Patent Literature 1: Fujiwara, T. et al. Curr Cancer Drug Targets, 7: 191-201, 2007.
Non Patent Literature 2: Lawrence et al., Gastroenterology Research and Practice, vol. 2013, (2013-01-01), pages 1-3
Non Patent Literature 3: Kagawa, S. et al., Annual Meeting of Japan Society of Clinical Oncology, vol. 53 , pages 1-4
Non Patent Literature 4: Koujima et al., Cancer Research, vol. 75, no. 15, 1 August 2015 (2015-08-01), pages 1-4

### Summary

Under the aforementioned circumstances, it has been desired to develop a method for treating tumor, by which the effects of OBP-301 are more strongly exhibited. In addition, it has also been desired to develop a method of administering an oncolytic virus to gastrointestinal cancers, in particular, gastrointestinal cancers located far from the esophagus, without performing surgical operations, which thereby imposes less burden on patients.

### Solution to Problem

As a result of intensive studies directed towards achieving the aforementioned objects, the present inventor has succeeded in treating a tumor by directly administering OBP-301 to tumor tissues under an endoscope, thereby completing the present invention.

Specifically, the present invention is a virus for use in a method of treating a tumor using an endoscope, wherein the method comprises inserting an endoscopic puncture needle filled with a virus-containing solution into tumor tissues and injecting the virus into the tumor tissues, and wherein the virus is administered to the peripheral base of the tumor and/or the marginal region of the tumor tissues, and wherein the virus is an oncolytic virus. The invention is defined in the appended claims.

According to the present invention, it has become possible to directly administer an oncolytic virus to tumor tissues under an endoscope and to treat the tumor.

### Brief Description of Drawings

Figure 1 is a view showing a method of filling an endoscopic puncture needle with a drug solution.
Figure 2 is a view showing the sites of cancer tissues, to which OBP-301 is administered.
Figure 3 is a view showing the sites of cancer tissues, to which OBP-301 is administered.
Figure 4 is a view showing a method of administering OBP-301 to cancer tissues.
Figure 5 is a view showing a method of administering OBP-301 to cancer tissues.
Figure 6 is a view showing a case process when a patient with esophageal cancer was treated with OBP-301.
Figure 7 is a view showing a case process when a patient with esophageal cancer was treated with OBP-301.
Figure 8 is a view showing a case process when a patient with esophageal cancer was treated with OBP-301.
Figure 9 is a view showing a case process when a patient with esophageal cancer was treated with OBP-301.

The present invention relates to a method of endoscopically administering a virus to tumor tissues. Specifically, the invention provides a virus for use in a method of treating a tumor using an endoscope, wherein the method comprises inserting an endoscopic puncture needle filled with a virus-containing solution into tumor tissues and injecting the virus into the tumor tissues, and wherein the virus is administered to the peripheral base of the tumor and/or the marginal region of the tumor tissues, and wherein the virus is an oncolytic virus. In one embodiment of the present invention, the virus includes adenovirus, herpesvirus, vesicular stomatitis virus, reovirus, vaccinia virus, and measles virus. Among these viruses, adenovirus, herpesvirus, and vesicular stomatitis virus are preferable, and adenovirus is particularly preferable. In the present invention, an adenovirus containing an hTERT promoter in the genome thereof is preferable.

In a preferred embodiment of the present invention, the oncolytic virus includes a recombinant oncolytic virus. The recombinant oncolytic virus of the present invention (for example, OBP-301) means a virus, in which a polynucleotide comprising a human telomerase promoter (hTERT promoter), an E1A gene, an IRES sequence and an E1B gene in this order is incorporated into the genome thereof. The type of the oncolytic virus used in the present invention is not particularly limited, and from the viewpoint of safety, adenovirus is preferable. Among such adenoviruses, adenovirus type 5 is particularly preferable because of its easy handlability. The recombinant oncolytic adenovirus can be obtained by the method described in WO2004/005511. Otherwise, OBP-301 as a recombinant oncolytic adenovirus can be acquired as "Telomelysin" (registered trademark) from Oncolys BioPharma Inc. Moreover, OBP-702, in which a polynucleotide comprising an Egr-1 promoter and a p53 gene in this order is incorporated into the E3 region of OBP-301, can also be preferably used. OBP-702 can be acquired from Oncolys BioPharma Inc.

The term "cancer" is used herein to mean a malignant tumor understood in the present technical field. For example, cancer has a property by which it proliferates, without being controlled, in tissues that are likely to spread to the remote sites of the body (i.e., to metastasize). Examples of such malignant tumor (cancer) to be administered with a virus may include, not only stomach cancer and gastroesophageal junction cancer, but may also include esophageal cancer, duodenal cancer, pancreatic cancer, colon cancer, head and neck cancer, anal cancer, rectal cancer, small intestine cancer, lung cancer, and liver cancer. The head and neck cancer includes laryngeal cancer and pharyngeal cancer. Among these cancers, stomach cancer, gastroesophageal junction cancer, duodenal cancer, small intestine cancer, colon cancer, anal cancer, and rectal cancer are desirable.

The oncolytic virus of the present invention (for example, OBP-301) can be directly administered to cancer tissues via an endoscope every two weeks. For example, in a radiation therapy period for 6 weeks, OBP-301 can be endoscopically directly administered into cancer tissues a total of three times, namely, on Day 1, Day 18, and Day 32. Hereafter, the case of using OBP-301 as an oncolytic virus will be described for explanatory convenience, but a person skilled in the art could readily apply other viruses as well.

### 1. Preparation of OBP-301 for use in injection

During the transportation and storage of OBP-301, the temperature is maintained at -60°C or lower.

At a stage in which OBP-301 is ready to be administered, a box is removed from a refrigerator, and a vial is then removed from the box. Subsequently, a cryopreserved OBP-301 solution (also referred to as an "IP solution") is thawed at room temperature. The time required for thawing the IP solution is approximately 10 minutes. After completion of the thawing, the IP solution is stable for 4 hours. If necessary, the IP solution can be preserved in ice before injection. The IP solution is filled in a single vial, so that 2 mL of the IP solution having a concentration of 1 x 10¹² VP/mL can be collected from the vial. Accordingly, a required amount of the IP solution is prepared, depending on the size of a lesion and the void volume to be filled with the IP solution.

### 2. Device for administering IP solution into tumor tissues

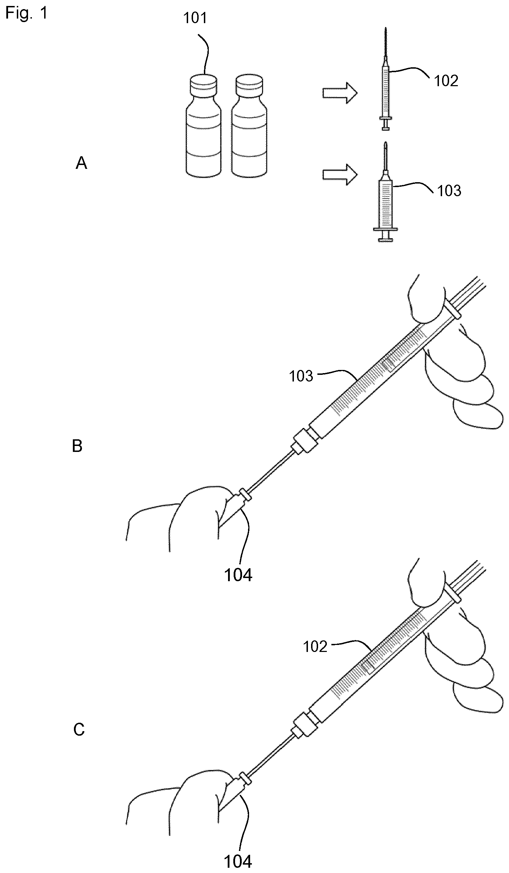

Figure 1 is a view showing vials and syringes at a preparation stage for administration of the IP solution into tumor tissues.

In Figure 1A, one to three vial(s) 101 (two vials are shown in the figure), a 1-mL syringe 102, and a 2.5-mL syringe 103 are prepared. The syringe 102 is a syringe for injecting the IP solution into tumor tissues, whereas the syringe 103 is a syringe for filling the IP solution into a tube connected with an endoscopic puncture needle (not shown in the figure).

In the present invention, in addition to the one to three vial(s) 101 comprising OBP-301, a set of endoscopic puncture needles, for example, Carr-Locke injection needles (sheath diameter: 2.5 mm, length: 230 cm, needle protrusion: 5 mm, and 25 gauge), or TOP endoscopic puncture needles for esophagus (length: 1600 mm, needle protrusion: 4 mm, and 23 gauge) are prepared. Thereafter, using the syringe 103, the IP solution is removed from the vial 101. The endoscope to be used can be selected, as appropriate, depending on the type of cancer. For example, a gastrointestinal endoscope, an airway endoscope, an ultrasonic endoscope, etc. can be adopted.

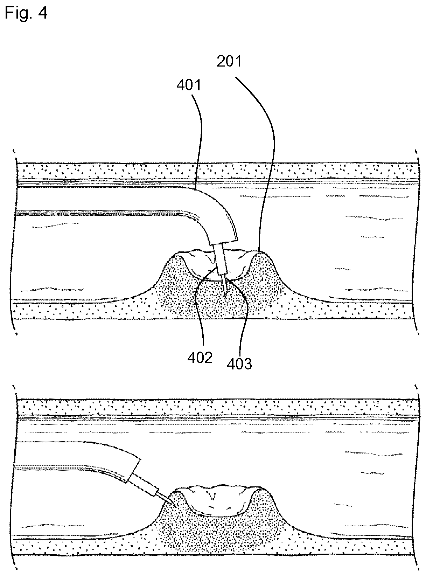

The IP solution is filled into an endoscopic puncture needle, specifically, into an endoscope tube 104 connected with the endoscopic puncture needle, immediately before the administration thereof. In the present invention, for explanatory convenience, not only an endoscopic puncture needle itself, but also an endoscopic puncture needle 403 connected with the endoscope tube 104 (see Figure 4), are collectively referred to as, simply, an "endoscopic puncture needle." The 2.5-mL syringe 103 containing the IP solution is attached to the end of the endoscopic puncture needle used, and then, the IP solution is slowly pushed out, so that the IP solution is filled into the endoscope tube 104 as a whole (Figure 1B). During this operation, attention is paid not to leak the IP solution from the tip of the puncture needle. After completion of the filling, the endoscope tube 104 filled with the IP solution is gently placed on a treatment table, while not pushing the syringe. Thereby, preparation of the IP solution to be administered is completed. When the IP solution is administered into tumor tissues, the syringe 103 is removed in order to administer the IP solution contained in the endoscope tube 104 into the tumor tissues, and the syringe 102 filled with the IP solution is then attached to the tube, and thereafter, the IP solution contained in the syringe 102 is pushed out of the end thereof (Figure 1C). Thereby, the IP solution filled in the endoscope tube 104 can be administered at a maximum dose of 1 mL (for example, 0.2 ml x 5 times of administrations). The IP solution to be administered into tumor tissues is filled in the tube. A solution used to push the IP solution into the tumor tissues is not limited to the IP solution, and for example, a normal saline or the like can be used. However, from the viewpoint of reducing the risk of diluting the IP solution to be administered into tumor tissues with such a normal saline, the IP solution is preferably used also as a solution used to push the IP solution into the tumor tissues.

### 3. Method of administering IP solution into tumor tissues and method for treating tumor

Next, the method of administering the IP solution into tumor tissues will be described. In the present description, type 2 esophageal cancer is exemplified and explained. First, the cancer region as a whole is observed, and administration sites are then determined.

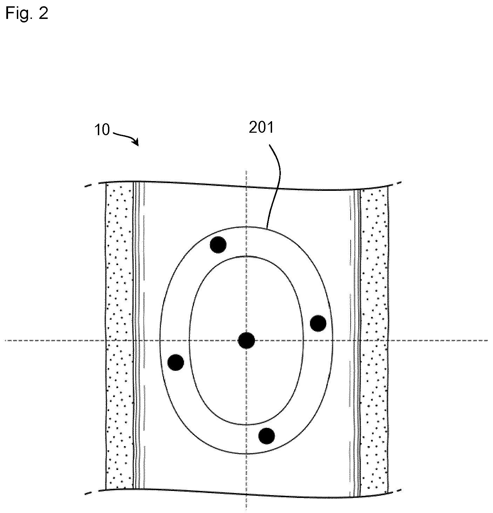

Figure 2 is a view showing the positions of tumor tissues 201 of esophagus 10, into which the IP solution is to be administered. In order that the IP solution can cover the entire region, the IP solution can be administered to at least 5 administration sites (for example, 5 to 10 sites) in the administration region, depending on the size of the tumor. In Figure 2, the administration sites are indicated with the filled circles.

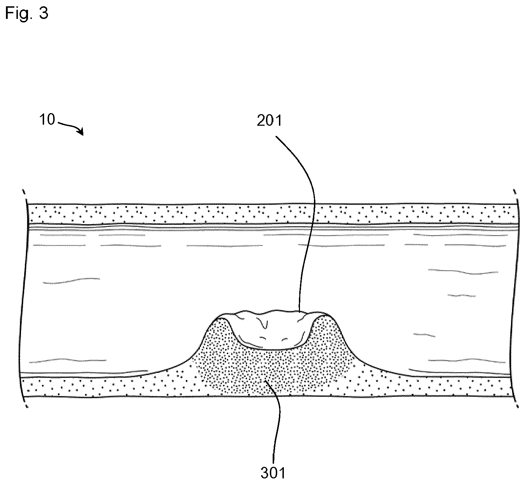

Upon administration of the IP solution into tumor tissues, iodine staining is not carried out in order to prevent inactivation of the IP solution. In addition, the lesion of the tumor may be observed as necessary, and administration sites may be then determined using image enhancement imaging (Figure 3). Figure 3 shows that the image of an administration region 301 was enhanced by the image enhancement imaging in an esophagus 10 having tumor tissues 201.

Figure 4 is a view showing a state in which the IP solution is injected into tumor tissues 201 from an endoscopic puncture needle 403. In Figure 4, an endoscope 401 is allowed to reach the tumor tissues 201 of the esophagus 10. After confirmation of a lesion, the endoscopic puncture needle 403 connected with an endoscope tube 402 is inserted into the lesion from a forceps channel, and the IP solution used as a drug solution is then injected, so that the IP solution can be spread over the entire tumor, successively from the tumor margin on the tail side to the tumor margin on the mouth side. This operation is carried out to prevent the phenomenon in which the administration sites become invisible due to bleeding occurring upon the administration. The IP solution is preferably administered to sites in which cancer cells actively divide, and thus, the IP solution is preferably injected into the peripheral base of the tumor or the marginal portion of the tumor. Moreover, administration of the IP solution to sites in which necrosis may occur is preferably avoided.

The IP solution is administered with good balance to at least 5 lesion sites (administration sites) at a predetermined dose per site. When the IP solution is injected into the lesions, a 1-mL syringe is used. While a person in charge of injection operations confirms the syringe scale, the person slowly injects the IP solution over approximately 5 seconds per site. In the present invention, the amount of the IP solution is preferably 0.1 mL or more, more preferably 0.15 mL or more, and particularly preferably 0.2 mL or more. The administration time is not limited, and administration is carried out preferably for 5 seconds or more per site, and more preferably for 5 seconds per site. According to the repeated administrations on Day 18 and Day 32, it may become impossible to identify the cancer due to reduction of the cancer. In this case, administration is carried out on the same administration sites as those on Day 1.

Next, an administration method applied in the case of esophagostenosis or superficial cancer of esophagus will be described.

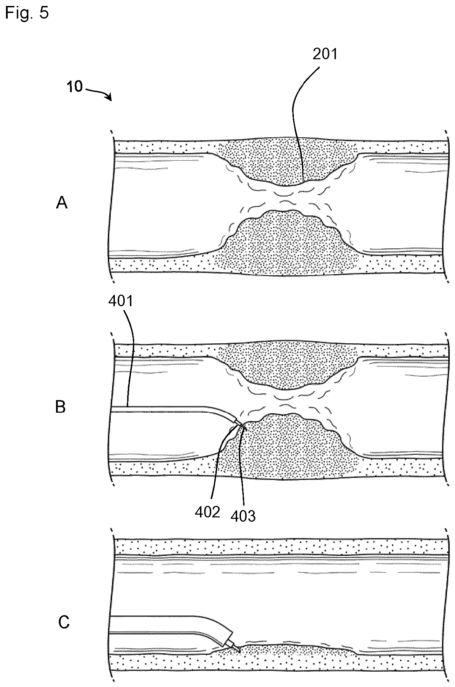

Figure 5 is a view showing that an esophagus 10 had esophagostenosis due to tumor tissues 201. When a normal endoscope hardly passes through the esophagus due to such esophagostenosis (Figure 5A), a transnasal endoscope is used as necessary, and in principle, 0.2 mL of the IP solution is administered to 5 or more sites. In the case of superficial cancer of esophagus or small elevated cancer, an endoscope 401, an endoscope tube 402, and an endoscopic puncture needle 403 are placed as horizontal as possible, and intratumoral administration is then carried out (Figures 5B and C). Furthermore, submucosal injection of a normal saline for upraising cancer is preferably not carried out because the drug is diluted with the normal saline in some cases.

As mentioned above, cancer can be treated by administering a virus to a patient according to the above-described method.

### Examples

### [Example 1]

### 1. Method

Patients with esophageal cancer, to whom standard treatments (surgical resection and chemotherapy) were hardly applied, were used as subjects, and an administration test of OBP-301 (Telomelysin) was carried out.

The backgrounds of the patients are shown in Table 1 below.

**[Table 1]**

| Cohort | ID | Age | TMN or Stage | Histological Type |
|---|---|---|---|---|
| Cohort 1 | 001 | 82 | T1bN0M0 (Stage IA) | Squamous cell carcinoma |
| | 002 | 84 | T3N1M0 (Stage IIIA) | Adenocarcinoma |
| | 003 | 82 | T1bN1M0 (Stage IIB) | Squamous cell carcinoma |
| Cohort 2 | 004 | 79 | T1bN0M0 (Stage IA) | Squamous cell carcinoma |
| | 005 | 66 | T3N1M0 (Stage IIIA) | Squamous cell carcinoma |
| | 006 | 85 | T2N0M0 | Squamous cell |
| | | | (Stage IB) | carcinoma |

The applied dose of OBP-301 was set to be 1 x 10¹¹VP/mL for Cohort 1, and 1 x 10¹²VP/mL for Cohort 2. In each cohort, on Day 1, Telomelysin was administered, at a dose of 0.2 ml to a single lesion of thoracic esophagus, to 5 sites (a total of 1 ml), using an endoscope under local anesthesia. Thereafter, intratumoral additional administration of Telomelysin was carried out on Day 18 and on Day 32. It is to be noted that, in the present example, from Day 4, radiation exposure was carried out at a dose of 2.0 Gy/day, 5 times a week (10Gy/week), for 6 weeks (total radiation dose: 60 Gy).

The treatment period was set to be 6 weeks, and evaluation was carried out with regard to the following evaluation items.
(1) Primary evaluation items:
   - Occurrence rate of dose-limiting toxicity (DLT)
   - Occurrence rate of harmful phenomenon
(2) Secondary evaluation items:
   - Effect of reducing tumor in therapeutic target lesion from initiation of treatment until 18th week (topical treatment effect)
   - Effect of reducing tumor from initiation of treatment until 18th week (systemic treatment effect, Recist evaluation)

### Results

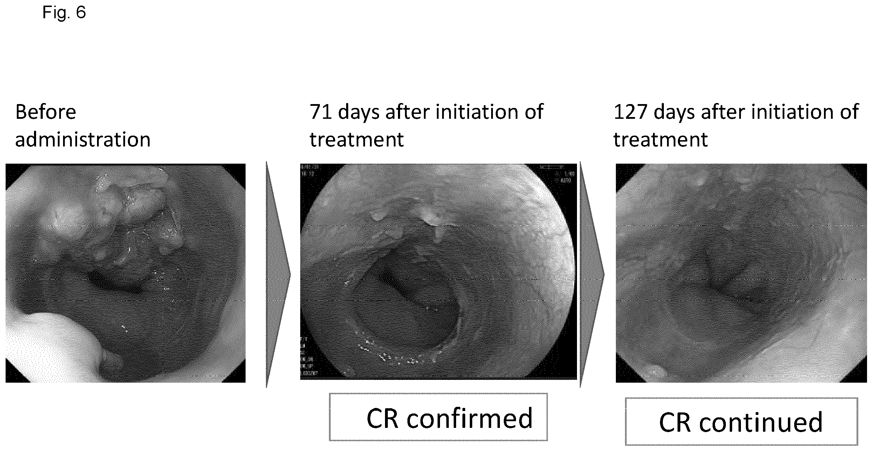

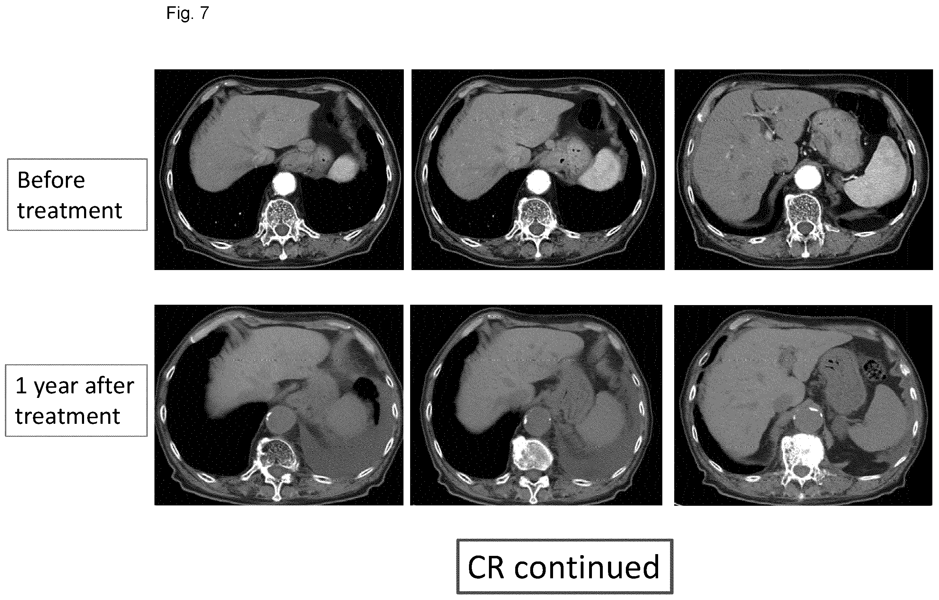

The progress of the case with ID number of 002 (Cohort 1) is shown in Figure 6 (photographs of esophageal portion) and Figure 7 (CT images). On 71 day after initiation of the treatment, the patient achieved a complete response (CR), and even on Day 127 after initiation of the treatment, the CR continued (Figure 6). In addition, 1 year after the treatment, lymph node metastasis disappeared, and the CR still continued (Figure 7).

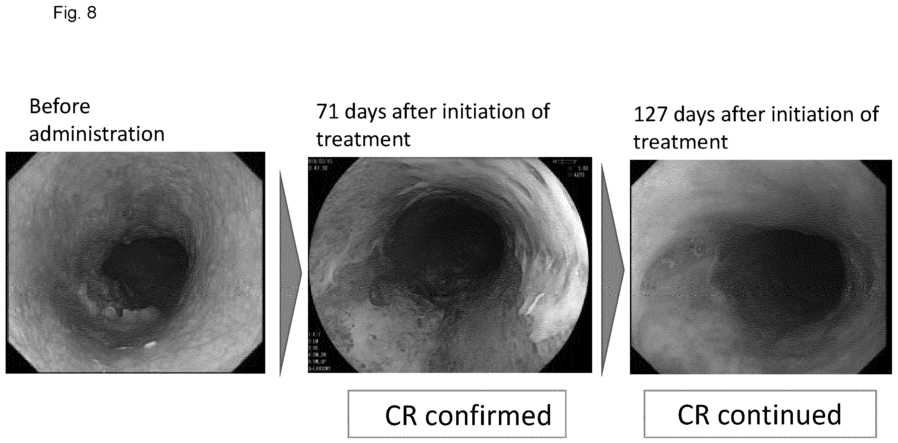

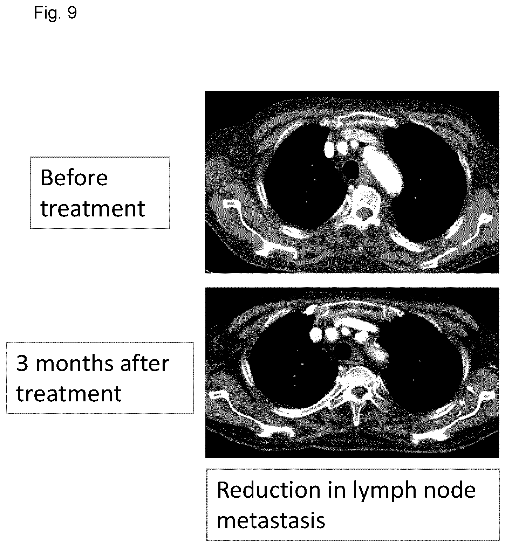

The progress of the case with ID number of 003 (Cohort 1) is shown in Figure 8 (photographs of esophageal portion) and Figure 9 (CT images). Also, in this case, CR was confirmed on 71 day after initiation of the treatment (Figure 8), and even on Day 127 after initiation of the treatment, the CR continued (Figure 8). In addition, 3 months after the treatment, lymph node metastasis was reduced (Figure 9).

### Description of Symbols

10: Esophagus
101: Vial, 102: 1-mL Syringe , 103: 2.5-mLSyringe , 104: Tube
201: Tumor tissues
301: Administration region
401: Endoscope, 402: Endoscope tube , 403: Endoscopic puncture needle

## Claims

1. A virus for use in a method of treating a tumor using an endoscope,
wherein the method comprises inserting an endoscopic puncture needle filled with a virus-containing solution into tumor tissues and injecting the virus into the tumor tissues,
wherein the virus is administered to the peripheral base of the tumor and/or the marginal region of the tumor tissues, and
wherein the virus is an oncolytic virus.

2. The virus for use according to claim 1, wherein the tumor is at least one selected from the group consisting of esophageal cancer, gastroesophageal junction cancer, stomach cancer, anal cancer, rectal cancer, colon cancer, head and neck cancer, duodenal cancer, pancreatic cancer, small intestine cancer, lung cancer, and liver cancer.

3. The virus for use according to claim 1, wherein the virus is administered to at least 5 sites in the tumor tissues.

4. The virus for use according to claim 1, wherein the virus is administered in an amount of at least 0.1 mL to 1 site in the tumor tissues.

5. The virus for use according to claim 1, wherein the virus is administered over at least 5 seconds to 1 site in the tumor tissues.

6. The virus for use according to claim 1, wherein the virus is additionally administered to the same lesion one or more times.

7. The virus for use according to claim 1, wherein the virus is selected from the group consisting of an adenovirus, a herpesvirus, and a vesicular stomatitis virus.

8. The virus for use according to claim 1, wherein the virus is an adenovirus containing an hTERT promoter.

## Patentansprüche

1. Virus zur Verwendung in einem Verfahren zur Behandlung eines Tumors unter Verwendung eines Endoskops,
wobei das Verfahren das Einführen einer mit einer virushaltigen Lösung gefüllten endoskopischen Punktionsnadel in Tumorgewebe und das Injizieren des Virus in das Tumorgewebe umfasst,
wobei das Virus an die periphere Basis des Tumors und/oder in den Randbereich des Tumorgewebes verabreicht wird, und
wobei es sich bei dem Virus um ein onkolytisches Virus handelt.

2. Virus zur Verwendung nach Anspruch 1, wobei der Tumor mindestens einer ist, ausgewählt aus der Gruppe bestehend aus Speiseröhrenkrebs, Krebs des gastroösophagealen Übergangs, Magenkrebs, Analkrebs, Rektumkrebs, Dickdarmkrebs, Kopf-Hals-Krebs, Zwölffingerdarmkrebs, Bauchspeicheldrüsenkrebs, Dünndarmkrebs, Lungenkrebs und Leberkrebs.

3. Virus zur Verwendung nach Anspruch 1, wobei das Virus an mindestens 5 Stellen im Tumorgewebe verabreicht wird.

4. Virus zur Verwendung nach Anspruch 1, wobei das Virus in einer Menge von mindestens 0,1 ml an einer Stelle im Tumorgewebe verabreicht wird.

5. Virus zur Verwendung nach Anspruch 1, wobei das Virus über einen Zeitraum von mindestens 5 Sekunden an einer Stelle im Tumorgewebe verabreicht wird.

6. Virus zur Verwendung nach Anspruch 1, wobei das Virus zusätzlich ein- oder mehrmals an derselben Läsion verabreicht wird.

7. Virus zur Verwendung nach Anspruch 1, wobei das Virus ausgewählt ist aus der Gruppe bestehend aus einem Adenovirus, einem Herpesvirus und einem Vesikulären Stomatitis-Virus.

8. Virus zur Verwendung nach Anspruch 1, wobei das Virus ein Adenovirus ist, das einen hTERT-Promotor enthält.

## Revendications

1. Virus destiné à être utilisé dans une méthode de traitement d'une tumeur au moyen d'un endoscope,
la méthode comprenant l'insertion d'une aiguille de ponction endoscopique remplie d'une solution contenant le virus dans les tissus tumoraux et l'injection du virus dans les tissus tumoraux,
le virus étant administré à la base périphérique de la tumeur et/ou dans la région marginale des tissus tumoraux, et
le virus étant un virus oncolytique.

2. Virus destiné à être utilisé selon la revendication 1, la tumeur étant au moins une tumeur appartenant au groupe constitué par le cancer de l'œsophage, le cancer de la jonction gastroœsophagienne, le cancer de l'estomac, le cancer de l'anus, le cancer du rectum, le cancer du côlon, le cancer de la tête et du cou, le cancer du duodénum, le cancer du pancréas, le cancer de l'intestin grêle, le cancer du poumon et le cancer du foie.

3. Virus destiné à être utilisé selon la revendication 1, le virus étant administré à au moins 5 sites dans les tissus tumoraux.

4. Virus destiné à être utilisé selon la revendication 1, le virus étant administré en une quantité d'au moins 0,1 mL à 1 site dans les tissus tumoraux.

5. Virus destiné à être utilisé selon la revendication 1, le virus étant administré pendant au moins 5 secondes à 1 site dans les tissus tumoraux.

6. Virus destiné à être utilisé selon la revendication 1, le virus étant également administré à la même lésion une ou plusieurs fois.

7. Virus destiné à être utilisé selon la revendication 1, le virus étant choisi dans le groupe constitué d'un adénovirus, d'un herpèsvirus et d'un virus de la stomatite vésiculaire.

8. Virus destiné à être utilisé selon la revendication 1, le virus étant un adénovirus contenant un promoteur hTERT.
